# EUROPEAN PATENT SPECIFICATION

(11) **EP 1 545 314 B1**
(45) Date of publication and mention of the grant of the patent: **07.09.2016**
(21) Application number: 03763254.4
(22) Date of filing: 03.07.2003
(51) Int. Cl.: A61B 8/00, A61B 8/12, A61B 8/14, A61B 17/20, A61H 1/00, A61H 1/02, A61H 5/00

(54) **CARDIAC ABLATION USING MICROBUBBLES**
HERZABLATION MIT MIKROBLÄSCHEN
ABLATION CARDIAQUE FAISANT APPEL A DES MICROBULLES

(30) Priority: 08.07.2002 US 394392 P
(43) Date of publication of application: 29.06.2005
(73) Proprietor: Koninklijke Philips N.V., 5656 AE Eindhoven (NL)
(72) Inventor: LOPATH, Patrick, David, Stamford, CT 06902 (US)
(74) Representative: Damen, Daniel Martijn
(86) International application number: PCT/US2003/021140
(87) International publication number: WO 2004/004572

(56) References cited:
- WO-A-01/10313
- WO-A1-01/97698
- US-A- 5 558 092
- US-B1- 6 210 393
- US-B1- 6 312 402

## Description

### Cross-Reference to Related Applications

The present application claims benefit of U.S. Provisional Patent Application Serial No. 60/394,392, the disclosure of which is incorporated by reference herein.

### Technical Field

The present invention relates to medical procedures and devices for cardiac ablation and further relates to performing ultrasonic cardiac ablation, or similar medical procedures, with microbubbles.

### Background Art

Certain diseases can be treated by ablating tissues within the heart or in the vascular structures connected to the heart. Contraction or "beating" of the heart is controlled by electrical impulses generated at nodes within the heart and transmitted along conductive pathways extending within the wall of the heart. Diseases of the heart known as cardiac arrhythmias involve abnormal generation or conduction of the electrical impulses. One such arrhythmia is atrial fibrillation or "AF". Certain cardiac arrhythmias can be treated by deliberately damaging the tissue of the cardiac wall along a path crossing a route of abnormal conduction.

Cardiac arrhythmias such as AF have been treated by a procedure referred to as the "maze" procedure, in which a scar is formed by cutting into the cardiac tissue of the atrium. The scar tissue forms a barrier to propagation of abnormal electrical signals. The surgical procedure is conducted so that the scar, and hence the barrier, isolates the focus of the arrhythmia. As described, for example, in co-pending, commonly assigned U.S. Patent Application Serial Nos. 09/815,863 (published as U.S. Application No. 20020002371), 09/905,227 (published as U.S. Application No. 20020065512) and 09/904,620 (published as U.S. Application No. 20030013968), it has been proposed to form a similar barrier by applying ultrasonic energy to the tissue of the heart wall so as to ablate the myocardial tissue along a loop extending around the site of the arrhythmia as, for example, extending around the ostium of a pulmonary vein in the case of AF. As described in co-pending, commonly assigned U.S. Patent Application Serial Nos. 09/904,963 (published as U.S. Application No. 20020068885), 10/062,693 (published as U.S. Application No. 20020115990) and 10/131,755 (published as U.S. Application No. 20030120270), AF also can be treated by ablating myocardial tissue which extends from the heart wall along the pulmonary vein.

The medical procedure conducted to generate a scar can be performed by conventional surgery, but this entails all of the risks and expense associated with cardiac surgery. As described in *Lesh,* International Publication WO 99/02096, , the abnormal conduction routes in AF typically extend from the wall of the heart along the pulmonary veins. Therefore, AF can be treated by ablating tissue in a ring around each pulmonary vein at the juncture between the pulmonary vein and the heart. As described in the '096 publication, such ablation can be performed by threading a catheter having a thermal ablation element at its distal tip into the heart so that the tip is lodged within the appropriate pulmonary vein. The catheter may bear a balloon which is inflated within the vein and which holds the catheter in place. The ablating element is then actuated so as to apply heat in a region surrounding the ablating element. In certain embodiments taught in the '096 publication, the ablating element includes a radio frequency ("RF") emitting element which is carried on the surface of the balloon. RF energy applied through the electrode heats the tissue to a degree sufficient to cause death of the normal tissue and its replacement by scar tissue. Heating to this degree is referred to herein as "ablation." Typically, heating to about 60-80°C is sufficient. Ablation of the pulmonary vein using RF energy can create a rough, disrupted surface on the interior of the vein. This or other factors can lead to stenosis of the pulmonary vein or thrombosis, i.e., formation of blood clots.

Another approach, described in Swartz et al., U.S. Patent 5,575,766, is to introduce a catheter bearing a localized energy emitter such as an electrode for application of RF energy at its distal tip into a heart chamber, such as the right or left atrium of the heart in the case of AF. The physician then moves the catheter so that the tip, and the localized emitter traces the desired path. In AF, the desired path typically is a closed loop encircling the openings or ostia of the pulmonary veins. Tracing a precise path along the interior of a chamber in the heart of a living subject with the tip of a catheter involves inherent practical difficulties. Although curved guide wires can be placed within the catheter so that the catheter tip will tend to follow the guide wire as the physician moves it, the process is still difficult.

The last few years have seen the introduction of microbubbles as contrast medium for use in diagnostic ultrasound imaging. These bubbles are used because they are highly attenuative, and serve to highlight vascular and highly perfused structures on brightness mode images. Many laboratories are now exploring various therapeutic uses for mechanically introduced microbubbles. With these developing avenues of research has come a host of engineered bubbles, of various sizes, gaseous inclusions and shell materials. Among other things, one of the parameters that bubble designers are able to control is the half-life of these bubbles within the body before they dissolve.

In work unrelated to treatment of cardiac arrhythmias, Unger, U.S. Patent 6,088,613, describes a method of magnetic resonance imaging focused surgical and therapeutic ultrasound in conjunction with the administration of a contrast medium. The contrast medium comprising gas filled vesicles is used in conjunction with magnetic resonance imaging (MRI) on a patient requiring surgery, using the contrast medium when conducting the MRI scan on the patient to identify the region of the patient requiring surgery, and then applying ultrasound to the region identified.

Ultrasonic ablation within the heart and in the vascular structures associated with the heart has major advantages over competitive techniques such as RF ablation. Among these advantages are the significantly greater attenuation of ultrasound energy in tissue than in blood, (unlike RF) allowing tissue heating without risk of blood coagulation, and the ability of ultrasound to deposit energy into tissue at depth, heating it directly, rather that relying on thermal conductivity as RF ablation does. While these advantages are significant, improvements to the conventional use of high intensity ultrasound are still possible. One of the pitfalls of any energy modality in the body is the inability to target specific tissue types. The wavelength of the ultrasound energy typically used within the body allows the energy to be focused as described in the aforementioned co-pending, commonly assigned applications, which greatly improves anatomical targeting. Techniques using focused ultrasonic energy are commonly referred to by the acronym HIFU, for high-intensity focused ultrasound.

Tissue at the focal location is heated to a greater extent than tissue at other locations. Even with these improvements, however, it would still be desirable to reduce the length of time required to achieve the transmural lesions often required for cardiac ablations, and to further reduce the risk of damage to adjacent structures.

US 6,312,402 discloses catheters for improving blood flow to a patient's heart. The catheters use an ultrasound transducer to create revascularization channels and/or stimulation of angiogenesis in a patient's heart. In particular, ultrasound is used to mix, deliver and/or activate a medicament. The catheter of US 6,312,402, however, is not used to ablate heart tissue.

### Disclosure of the Invention

The present invention is defined by the appended independent claim. Preferred embodiments of the invention are defied in the dependent claims.
One non-claimed aspect of the present invention provides a method for ablating myocardial tissue within the wall of the heart or within the wall of a blood vessel connected to the heart. In preferred methods according to this aspect of the invention, microbubbles are supplied to the circulatory system of a mammalian subject. The microbubbles perfuse the myocardial tissue of the subject. While the microbubbles are present in the myocardial tissue, ultrasonic energy is applied to the wall of the heart or to the wall of a blood vessel connected to the heart. The myocardial tissue having the microbubbles present therein is heated by the ultrasonic energy and the myocardial tissue is then ablated.

Methods according to this aspect of the invention can be applied and used for pulmonary vein isolation or ablation, the treatment of AF, and for common medical and surgical procedures involving ultrasound ablation of the heart or associated vascular structures.

Ultrasound attenuation of the myocardium is selectively increased with respect to the adjacent venous tissue and with respect to other adjacent structures by introducing microbubbles into the circulatory system so that the microbubbles enter the coronary arteries and pass into the myocardial tissue. The myocardium is highly perfused tissue, receiving its blood supply from the coronary arteries. An injection of microbubbles in the left ventricle ("LV") results in the movement of microbubbles into the coronary arteries through the aorta, and subsequently perfusion of the myocardium with the microbubbles. An injection of these bubbles into the left atrium ("LA") will also result in the movement of the microbubbles through the mitral valve into the LV, out of the LV via the aortic root, and into the coronary arteries. An injection into the aorta, for example, at the openings of the coronary arteries, or directly into a particular coronary artery, will provide even more immediate passage of the microbubbles into the myocardial tissue to be ablated, and will minimize dilution of the microbubbles.

Venous tissue is costly connective, and not directly or highly vascularized. Therefore, introduction of microbubbles into the blood passing to the heart via the coronary arteries increases the rate of ultrasonic absorption in myocardial tissues to a far greater degree than in venous tissue. As discussed above, certain techniques which have been proposed for treatment of AF direct ultrasonic energy into the wall of the pulmonary vein so as to ablate the myocardial muscular sheathes that extend up the pulmonary veins from the heart. By selectively increasing the rate of ultrasonic absorption of the myocardial tissue, introduction of microbubbles promotes selective heating of the muscular sheathes, and thus limits damage to the venous tissue. This, in turn, helps to avoid stenosis of the pulmonary veins resulting from the treatment. Stated another way, heating of the venous tissue can cause the vein to rapidly occlude. This is a potentially life threatening condition. The preferred techniques according to the present invention can reduce the risk of such vein stenosis.

Also, regardless of whether the ultrasonic energy is applied in the heart wall or in the vein wall, the enhanced absorption rate of the cardiac tissue allows faster ablation and reduces the total energy which must be delivered during the procedure, which in turn reduces the risk of collateral damage. Additionally, the enhanced absorptivity of the myocardial tissue reduces transmission of ultrasound through the cardiac wall to surrounding structures such as nerves, and thus further reduces the risk of collateral damage. Moreover, by reducing the total energy which must be delivered, the microbubbles reduce the waste heat dissipated by the ultrasonic applicator. Certain ultrasonic applicators disclosed in the applications mentioned above use a balloon filled with an aqueous liquid surrounding a piezoelectric ultrasonic applicator. The fluid is circulated into and out of the balloon to maintain the balloon at a temperature of the balloon below the level of coagulation of the blood, (60-65°C). By reducing the amount of energy which must be delivered, the need for such circulation can be reduced or eliminated, greatly simplifying the procedure and reducing the cost of the ablation system and catheter.

The timing of the high power sonication can be determined through the use of intracardiac or other ultrasound imaging. Currently, much of the pulmonary vein isolation procedure is guided by intracardiac ultrasound, for example, with an imaging catheter in the right atrium ("RA") looking toward the LA and the pulmonary veins. An imaging procedure optionally can be used in methods according to this aspect of the invention. As the myocardium of the LA is observed to brighten in the diagnostic image, indicating that the microbubbles have perfused the myocardium, the ultrasonic energy can be applied.

The microbubbles chosen for this type of selective ablation would ideally be short-lived, with an engineered half-life of less than one trip around the body. As is known in design of microbubbles for use as ultrasonic imaging contrast agents, the half-life of microbubbles is controlled by factors such as the composition of the material surrounding the gas in the microbubble, commonly referred to as the "shell" of the microbubble, and by the diffusion rate of the gas within the microbubble. For example, common microbubbles used as ultrasonic contrast agents contain carbon dioxide surrounded by a protein micelle. By designing bubbles to have the proper half-life, bubbles introduced to the arterial system before the ablation of one pulmonary vein would not be present in the LA (in the blood returning from the lungs) as the next sonication was delivered by an ablation device. Thus, the half-life of the microbubbles preferably should be selected so that it is sufficient to survive the relatively brief interval required for movement of the microbubbles from the site of administration (the LA, LV, aorta or coronary arteries) into the myocardial tissue but not sufficient to survive a complete passage through the systemic circulation, the heart and the pulmonary circulation back into the LA. Timing in this manner would maintain the advantage that ultrasound has in being able to penetrate blood relatively unabated, depositing energy only in the tissue.

A further aspect of the invention provides a kit for ablating myocardial tissue of a mammalian subject. The kit according to this aspect of the invention desirably includes an ultrasonic ablation device adapted to apply ultrasonic energy to the wall of the heart or to a blood vessel connected to the heart, together with microbubbles adapted for administration into the circulatory system of the subject. The ultrasonic ablation device may be carried on a catheter structure which extends into or through the LA or LV in operation. The catheter structure may include a lumen having an opening that communicates with the LA or LV that allows for microbubbles to be dispersed. Alternatively, the kit may include a separate microbubble catheter adapted for insertion into the heart, in the aorta, or into a coronary artery, at a position that is not in close proximity to the ultrasonic ablation device. The kit optionally may include an imaging device.

### Brief Description of Drawings

FIG. 1 is a diagrammatic view depicting one embodiment of the invention.
FIG. 2 is a diagrammatic view of a further embodiment of the invention.
FIG. 3 is a diagrammatic view of yet another embodiment of the invention.

### Best Mode for Currying Out the Invention

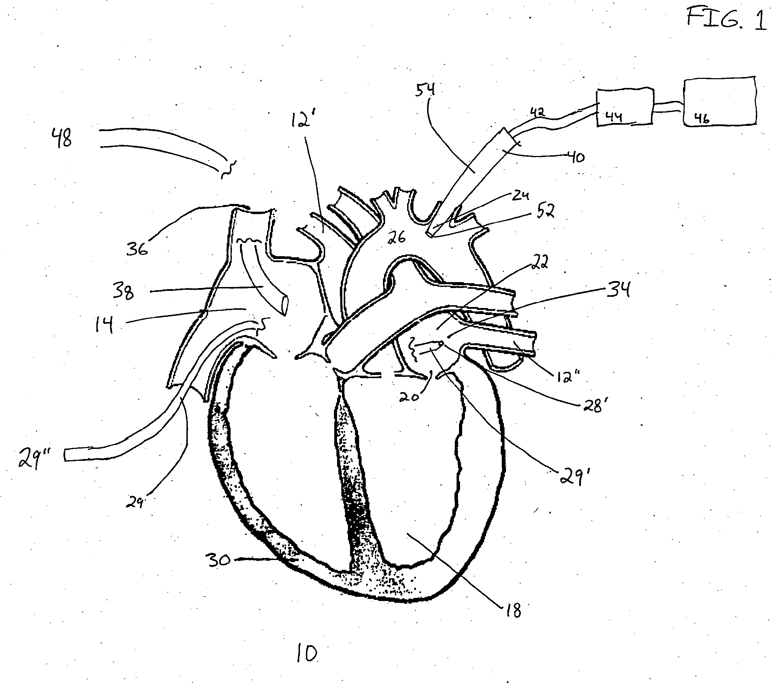

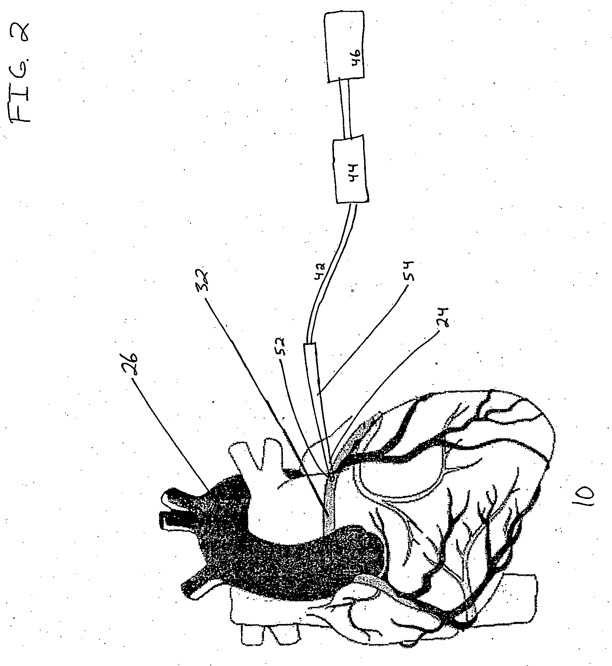

FIG. 1 shows a method for performing cardiac ablation in accordance with one embodiment of the present invention. The method includes the use of an ultrasonic ablation device 28. Device 28 preferably is a device according to the aforementioned co-pending, commonly assigned U.S. Patent Application Serial Nos. 09/905,227, 09/904,963 and 09/904,620, and includes an ultrasonic emitter together with a reflector or lens for focusing the energy emitted from the emitter onto a region of tissue to be ablated. Device 28 desirably is mounted on the distal end 29' of catheter referred to herein as the device catheter. The proximal end 29'' of the device catheter is connected to a source of excitation energy (not shown) from the ultrasonic device. The apparatus further includes a microbubble catheter 54, and an intracardiac or ultrasound imaging device 48. The distal end of the ultrasonic ablation device 28' is moved into the heart 10 by a physician. The physician then moves the distal end 29' of the device catheter so that the ultrasonic ablation device 28 is positioned in proximity to myocardial tissue to be ablated 34 in the left atrium 22, while the proximal end of the device catheter 29'', remains outside the patient's body. The distal end 24 of the microbubble catheter 54 is positioned in the aorta 26. The proximal end 40 of the microbubble catheter 54 is connected by tubing 42 to a pump 44. The pump 44 facilitates the movement of the microbubbles from a microbubble supply 46 to the microbubble catheter 54. Pump 44 may be integrated with microbubble supply 46. For example, the pump may be a manually-operated or mechanically-operated syringe. Typically the microbubbles are provided as a slurry in a liquid carrier acceptable for introducing into the body. Microbubbles enter the aorta 26 by exiting the lumen of the microbubble catheter 54 at a portal 52 at the distal end of the microbubble catheter 24. The natural blood flow of the heart 10 feeds the microbubbles into coronary arteries 32 (see FIG. 2), which subsequently feeds blood to the myocardial tissue 30.

An imaging catheter 38 is positioned in the right atrium 14 to look toward the left atrium 22 and pulmonary veins 12', 12''. The imaging catheter 38 may be positioned in the right atrium 14 via the superior vena cava 36, or in any position desired by the physician to observe a region of the heart or myocardial tissue to be ablated. In such a position, a site to be ablated within the left atrium 22 or pulmonary veins 12', 12'' may be scanned with the imaging catheter 38 and viewed by a physician with an intracardiac or other ultrasound imaging device 48. When perfused with microbubbles, the myocardium 30 of the left atrium 22 is observed to brighten on the imaging device 48. The presence of microbubbles in the region to be ablated 34 signals that the ultrasonic energy can be applied from the ultrasonic ablation device 28.

The physician actuates the energy source connected to the ultrasonic ablation device 28, so that the device emits energy which impinges on the tissue to be ablated.

The ultrasound attenuation of the myocardial tissue 30 is selectively increased with respect to the adjacent tissues by the microbubbles present in the myocardial tissue 30.

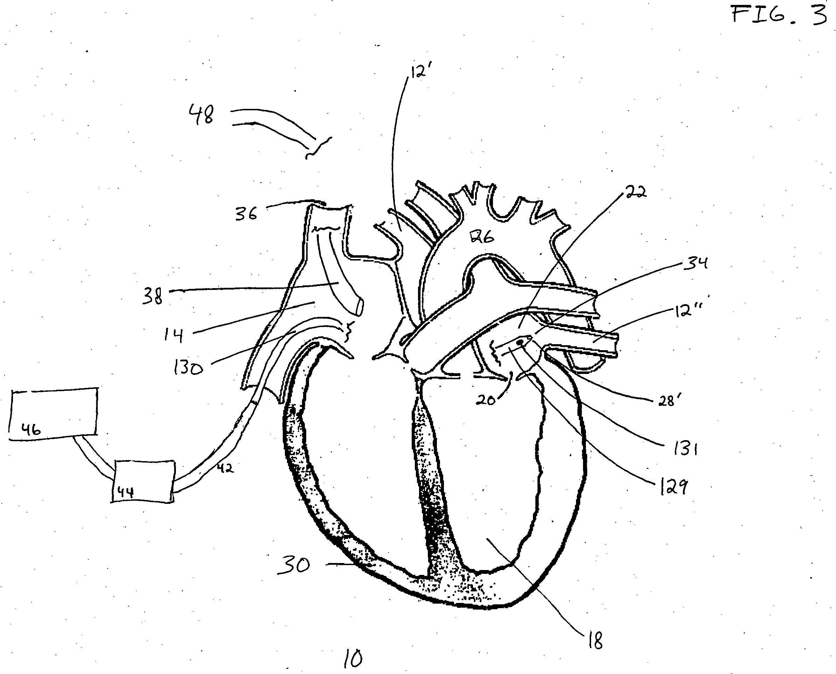

The apparatus and method according to the embodiment of FIG. 2 are the same as discussed above with reference to FIG. 1, except that the microbubbles are administered directly into a coronary artery 32. The myocardial tissue 30 is highly perfused, receiving its blood supply from the coronary arteries 32. In further variants of the invention, the microbubbles may be introduce into the circulatory system of the subject at a multiplicity of sites. For example, an injection of microbubbles in the left ventricle 18 results in the dispersion of microbubbles into the coronary arteries 32 via the aorta 26, and perfusion of the myocardium 30 with the microbubbles. An injection of microbubbles may also be performed into the left atrium 22 as discussed bellow with reference to FIG. 3.

The apparatus and method of FIG. 3 are the same as discussed above with perspective to FIG. 1, except that in the apparatus of FIG. 3, microbubble catheter structure 129 includes a lumen 130 with a port 131 adjacent to the distal end of the device catheter. Here again, the ultrasonic ablation device 28 is mounted adjacent to the distal end of the device catheter. Thus, when the physician positions the ablation device within the LA, adjacent to the tissue to be ablated, port 131 is also disposed in the LA. Lumen 130 is connected at the proximal end of the device catheter to a pump 44 and microbubble supply 46 as discussed above. Thus, in this embodiment, the device catheter performs the microbubble delivery function, and a separate microbubble catheter is not required. Release of microbubbles from the portal 131 of the microbubble catheter structure 129 deposits microbubbles into the left atrium 22. Natural blood flow moves the microbubbles through the mitral valve 20 and into the left ventricle 18. From the left ventricle 18 microbubbles enter the aorta 26, followed by entry into coronary arteries 32 (*see* FIG. 2) and then into myocardial tissue 30.

Microbubble composition and the composition of the shell of the microbubble affects the half-life of the microbubbles, which in turn affects the ultrasonic absorption of tissues containing the microbubbles. Preferably, during the ablation procedure, microbubbles are present in the tissue to be ablated but not in adjacent structures of the body that lie in the path of the ultrasonic energy. The presence of microbubbles in myocardial tissue increases the rate of ultrasonic absorption in myocardial tissues compared to other tissues, such as venous tissue. Preferably, the half-life of the microbubbles is short, with an engineered half-life of less than one trip around the body. Thus, the half-life of the administered microbubbles would be sufficient to survive the relatively brief interval required for movement from the site of administration (such as the left atrium 22, left ventricle 18, aorta 26, or coronary artery 32) into the myocardial tissue 30, but not sufficient to survive a complete travel through the systemic circulation of the body.

In a further variant of the procedure and apparatus discussed above, the imaging catheter 38 and imaging device 48 can be omitted. In this variant, the ultrasonic emitter is actuated at a predetermined time after administration of the microbubbles. This time is selected so that it corresponds to the time required for the microbubbles to pass from the site of administration to the tissue to be ablated.

A kit for performing the procedures discussed above desirably includes some or all of the apparatuses discussed above, together with the microbubbles.

### Industrial Applicability

The invention has applicability in the medical industry.

## Claims

1. A kit for ablating myocardial tissue of a mammalian subject comprising:
- an ultrasonic energy application device (28) that is carried on a device catheter and adapted to apply ultrasonic energy to the wall of the heart (10) or to a blood vessel connected to the heart (10); and
- microbubbles adapted for administration within the circulatory system of the subject;
**characterized in that** the kit further comprises:
- a catheter (54) that is separate from the device catheter and adapted for insertion into the aorta or into a cardiac artery for administering said microbubbles; wherein said microbubbles are adapted for selectively increasing the ultrasound attenuation of the wall of the heart (10) or the blood vessel connected to the heart (10).

2. A kit as claimed in claim 1, wherein said ultrasonic energy application device (28) is adapted to administer ultrasonic energy to the wall of a blood vessel connected to the heart (10).

3. A kit as claimed in claim 2, wherein said ultrasonic energy application device (28) is adapted to administer ultrasonic energy to the wall of a pulmonary vein (12', 12").

4. A kit as claimed in claim 1, wherein said ultrasonic energy application device (28) is adapted to administer ultrasonic energy to the wall of the heart (10).

5. A kit as claimed in claim 1, wherein said device catheter is configured to extend into or through the LA (22) or LV (18) when the device is in an operative condition.

## Patentansprüche

1. Kit zur Ablation von Myokardgewebe eines zu der Gattung der Säugetiere zählenden Patienten, wobei das Kit Folgendes umfasst:
- eine Ultraschallenergie-Zuführungsvorrichtung (28), die auf einem Vorrichtungskatheter getragen wird und dafür ausgelegt ist, der Wand des Herzens (10) oder einem mit dem Herz (10) verbundenen Blutgefäß Ultraschallenergie zuzuführen; und
- Mikrobläschen, die zur Verabreichung innerhalb des Kreislaufsystems des Patienten ausgelegt sind;
**dadurch gekennzeichnet, dass** das Kit weiterhin Folgendes umfasst:
- ein Katheter (54), das von dem Vorrichtungskatheter getrennt ist und zur Einführung in die Aorta oder in eine Herzarterie zur Verabreichung der genannten Mikrobläschen ausgelegt ist, wobei die genannten Mikrobläschen dafür ausgelegt sind, die Ultraschallabschwächung der Wand des Herzens (10) oder des mit dem Herz (10) verbundenen Blutgefäßes selektiv zu erhöhen.

2. Kit nach Anspruch 1, wobei die genannte Ultraschallenergie-Zuführungsvorrichtung (28) dafür ausgelegt ist, der Wand eines mit dem Herz (10) verbundenen Blutgefäßes Ultraschallenergie zuzuführen.

3. Kit nach Anspruch 2, wobei die genannte Ultraschallenergie-Zuführungsvorrichtung (28) dafür ausgelegt ist, der Wand einer Lungenvene (12', 12") Ultraschallenergie zuzuführen.

4. Kit nach Anspruch 1, wobei die genannte Ultraschall-Zuführungsvorrichtung (28) dafür ausgelegt ist, der Wand des Herzens (10) Ultraschallenergie zuzuführen.

5. Kit nach Anspruch 1, wobei der genannte Vorrichtungskatheter konfiguriert ist, um sich in der oder durch die LA (22) oder LV (18) zu erstrecken, wenn sich die Vorrichtung in einem Betriebszustand befindet.

## Revendications

1. Kit pour ablater un tissu myocardial d'un sujet mammifère, comprenant :
- un dispositif d'application d'énergie ultrasonique (28) qui est supporté sur un cathéter de dispositif et adapté pour appliquer de l'énergie ultrasonique sur la paroi du coeur (10) ou sur un vaisseau sanguin relié au coeur (10) ; et
- des microbulles adaptées pour l'administration à l'intérieur du système circulatoire du sujet ;
**caractérisé en ce que** le kit comprend en outre :
- un cathéter (54) qui est séparé du cathéter de dispositif et adapté pour l'insertion dans l'aorte ou dans une artère cardiaque pour administrer lesdites microbulles, dans lequel lesdites microbulles sont adaptées pour sélectivement augmenter l'atténuation ultrasonore de la paroi du coeur (10) ou du vaisseau sanguin relié au coeur (10).

2. Kit selon la revendication 1, dans lequel ledit dispositif d'application d'énergie ultrasonique (28) est adapté pour administrer de l'énergie ultrasonique sur la paroi d'un vaisseau sanguin relié au coeur (10).

3. Kit selon la revendication 2, dans lequel ledit dispositif d'application d'énergie ultrasonique (28) est adapté pour administrer de l'énergie ultrasonique sur la paroi d'une veine pulmonaire (12', 12").

4. Kit selon la revendication 1, dans lequel ledit dispositif d'application d'énergie ultrasonique (28) est adapté pour administrer de l'énergie ultrasonique sur la paroi du coeur (10).

5. Kit selon la revendication 1, dans lequel ledit cathéter de dispositif est configuré pour s'étendre dans ou à travers la LA (22) ou LV (18) lorsque le dispositif est dans une condition opérationnelle.
